# EUROPÄISCHE PATENTSCHRIFT

(11) **EP 0 654 462 B1**
(45) Veröffentlichungstag und Bekanntmachung des Hinweises auf die Patenterteilung: **22.04.1998**
(21) Anmeldenummer: 94117837.8
(22) Anmeldetag: 11.11.1994
(51) Int. Cl.: C07C 68/00, C07C 69/96

(54) **Verfahren zur Aufarbeitung der flüssigen Reaktionsprodukte aus der Cu-katalysierten Dimethylcarbonat-Herstellung**
Process for the treatment of liquid reaction products from the Cu-catalyzed production of dimethyle carbonate
Procédé de traitement des produits de réactions liquides provenant de la fabrication du carbonate de diméthyle catalysé par le cuivre

(30) Priorität: 24.11.1993 DE 4339977
(43) Veröffentlichungstag der Anmeldung: 24.05.1995
(73) Patentinhaber: BAYER AG, 51368 Leverkusen (DE)
(72) Erfinder: Rechner, Johann, Dr., D-47800 Krefeld (DE); Wagner, Paul, Dr., D-40597 Düsseldorf (DE); Buysch, Hans-Josef, Dr., D-47809 Krefeld (DE); Klausener, Alexander, Dr., D-50670 Köln (DE)

(56) Entgegenhaltungen:
- EP-A- 0 460 732
- DE-A- 3 926 709
- DE-B- 2 607 003

## Beschreibung

Die vorliegende Erfindung betrifft ein Verfahren zur Aufarbeitung flüssiger Reaktionsmischungen, wie sie bei der Dimethylcarbonat-Herstellung durch oxidative Carbonylierung von Methanol bei Anwesenheit eines kupferhaltigen Katalysators bei erhöhtem Druck und erhöhter Temperatur entstehen, das dadurch gekennzeichnet ist, daß man die nach der Abtrennung des Katalysators erhaltene Reaktionslösung durch einfache Destillation bei Normaldruck vom Reaktionswasser befreit, und das wasserfreie Kopfprodukt, bestehend aus Methanol und Dimethylcarbonat, durch eine Destillation bei erhöhtem Druck in reines Dimethylcarbonat als Sumpfprodukt und ein an Dimethylcarbonat abgereichertes Gemisch aus Methanol und Dimethylcarbonat als Kopfprodukt trennt, wovon das Kopfprodukt der Normaldruckkolonne oder der Reaktion wieder zugeführt wird.

In den vergangenen Jahren wurden eine Reihe von Verfahren zur Herstellung von Dialkylcarbonaten durch die katalytische Umsetzung der Edukte Methanol, Kohlenmonoxid und Sauerstoff entwickelt.

In DE-A 2 110 194 wurden als geeignete Katalysatoren Metallkomplexe der Gruppen IB, IIB und VIIIB des Periodensystems genannt, insbesondere die Metalle Cu, Ag, Au, Zn, Cd, Hg, Fe, Co und Ni, die bei Redoxreaktionen in zwei verschiedenen Oxidationsstufen existieren können. Dieses Verfahren liefert mit komplexiertem Cu₂Cl₂ gute Ausbeuten, hat aber den Nachteil, daß die Abtrennung der sehr teuren Komplexliganden und des gelösten komplexierten Katalysators von der Reaktionslösung aufwendig ist.

In DE-PS 2 743 690 werden statt der Kupferkomplexverbindungen einfache einwertige Salze des Kupfers als Katalysatoren verwendet. Diese Verfahrensvariante liefert zwar gute Ausbeuten an Dialkylcarbonaten, aber auch hier bereitet die Aufarbeitung der Reaktionslösung große Probleme, da der teilweise gelöste Katalysator von der Reaktionslösung abgetrennt werden muß. Nach der Lehre dieses Patents erfolgt dies durch Abfiltrieren des suspendierten Anteils und Rektifizierung oder Kristallisation des gelösten Katalysators. Da die katalysatorhaltigen Reaktionslösungen den Katalysator in weitere Anlagenteile tragen, ist für die Aufarbeitung der Reaktionslösung und des Katalysators ein hoher apparativer Aufwand erforderlich. Aufgrund der korrosiven Eigenschaften müssen alle Apparate (Kessel, Rohrleitungen, Destillations-, Kristallisations- und Filtrationsapparate), die mit dem Katalysator in Kontakt kommen, aus korrosionsbeständigem Material bestehen. Hierdurch verliert das Verfahren an Attraktivität.

Die gleichen Aufarbeitungsprobleme führen dazu, daß die Verwendung von Synthesegas statt CO, wie in DE-C 3 045 767 beschrieben, wirtschaftlich unattraktiv bleiben muß. Die durch den kupferhaltigen Katalysator verursachten Korrosionsprobleme bei der Aufarbeitung der Reaktionslösung lassen Verfahren, die weitere Zusätze zum Katalysator enthalten (z.B. EP-A 217 651, EP-B 090 977, US-A 4 370 275) unwirtschaftlich werden.

Eine verfahrenstechnische Alternative zur Abtrennung des Katalysators lehrt DE-OS 3 926 709. Hierbei verbleibt der kupferhaltige Katalysator im Reaktor. Das während der Reaktion gebildete Dialkylcarbonat wird zusammen mit dem Reaktionswasser und Methanol durch das Reaktionsgas aus der Reaktionsmischung gestrippt. Dieser Effekt wird im allgemeinen dadurch erzielt, daß ein Gasstrom von 20 bis 30 NL CO/O₂-Gasgemisch pro g im Reaktor als Kupferkatalysator vorhandenen Kupfers durch die Reaktionsmischung geleitet wird. Nachteilig bei diesem Verfahren sind die sehr großen Gasmengen, die im Kreislauf gehalten werden müssen und die hierdurch verursachten hohen Energiekosten sowie die Probleme bei der Gasdispergierung aufgrund der großen Gasmengen. Bei dieser Fahrweise muß zusätzlich die Temperatur und der Druck des Reaktors genau geregelt werden, um den Flüssigkeitsstand im Reaktor halten zu können, da bereits geringe Schwankungen der Reaktortemperatur oder des Druckes zu deutlich veränderten Austragsmengen führen. Für die in den EP 0 460 732 A1 und EP 0 460 735 A2 beschriebenen Verfahren gilt im wesentlichen das bei DE-A 3 926 709 Gesagte.

Im all diesen Verfahren wird zwar der Katalysator mehr oder weniger effektiv abgetrennt, aber die Verfahrensprobleme bei der Aufarbeitung der katalysatorfreien Reaktionslösung wurden auch bei diesen Anmeldungen nicht gelöst, d.h. die Wasserabtrennung aus der katalysatorfreien Reaktionslösung und die Trennung von Methanol und Dimethylcarbonat (DMC), da diese ein Azeotrop bilden.

Nach DE-OS 2450856 ist die Abtrennung des Dimethylcarbonats vom Reaktionswasser und Methanol (MeOH) mittels einer einfachen Rektifikation kompliziert, da sich verschiedene Azeotrope zwischen DMC, MeOH und Wasser bilden. Die Patentanmeldung lehrt eine Trennung durch Verwendung einer Extraktivdestillation unter Verwendung von Wasser als Lösungsmittel. Das Verfahren ist jedoch unwirtschaftlich, da erhebliche Wassermengen für die Trennung erforderlich sind (9,5 g Wasser auf 1 g Reaktionslösung).

Die DE-OS 2607003 beschreibt einen Trennversuch zur Spaltung des Methanol-Dimethylcarbonat-Azoetrops durch die Anwendung von Druck und erhöhter Temperatur. Man erhält nach der Lehre dieser Anmeldung zwar eine Bodenfraktion aus reinem DMC, bei dem Kopfprodukt handelt es sich jedoch erneut um eine Mischung aus Methanol und DMC, die an DMC verarmt ist. Wie ein solches Verfahrensfragment sinnvoll in ein Aufarbeitungsverfahren integriert werden kann, wird nicht gelehrt. Zusätzlich wird die Trennung ohne das bei der oxidativen Carbonylierung zwangsläufig anfallende Wasser durchgeführt, das, wie die DE-OS 3926709 lehrt, mit Methanol und Dimethylcarbonat ein ternäres Azeotrop bildet.

Die US 4 162 200 lehrt ebenfalls eine Extraktionsdestillation, wobei z.B.: Cyclohexan und Chlorbenzol als Hilfsmittel zum Einsatz kommen. Durch den Einsatz systemfremder Reagenzien werden zusätzliche Destillationsschritte erforderlich, die eine ökonomische Lösung des Trennproblems wieder verhindern. Zusätzlich können diese systemfremden Stoffe zu einer Verschmutzung des Dimethylcarbonats führen. Insbesondere ist bei der Anwendung von Chlorbenzol mit einer korrosiven Wirkung zu rechnen, was zu erheblich höheren Kosten führt.

In der US 3 803 201 wird ein Verfahren beschrieben, bei dem das Methanol-DMC-Azeotrop durch eine Kombination aus Tieftemperaturkristallisation, Filtration und anschließender fraktionierter Destillation aufgearbeitet wird. Allein schon durch die erforderliche Temperatur von ca. -70°C ist dieses Verfahren für eine technische Anwendung unbrauchbar. Die Anwendung von Kristallisation, Filtration und Destillationen stellt weiterhin einen erheblichen apparativen Aufwand dar, der das Verfahren unwirtschaftlich erscheinen läßt.

Es bestand nun die Aufgabe, ein einfaches und kostengünstiges Verfahren zur Aufarbeitung von Reaktionslösungen aus Dimethylcarbonat, Methanol und Wasser zu finden, wie sie bei der oxidativen Carbonylierung von Methanol mit Kupferchlorid als Katalysator entstehen.

Überraschenderweise wurde nun gefunden, daß man das Reaktionswasser und Dimethylcarbonat aus den Reaktionslösung, wie sie bei der oxidativen Carbonylierung von Methanol mit Kupferchlorid als Katalysator entstehen, statt durch umständliche Kristallisation, Filtration und Destillation oder durch Extraktivdestillation durch die Kombination einer Normaldruckdestillation mit einer Destillation bei erhöhtem Druck isolieren kann. Diese Kombination von Normaldruckdestillation zur Wasserabtrennung und anschließender Druckdestillation der wasserfreien Reaktionslösung zur Dimethylcarbonatisolierung und Rückführung des methanolischen Kopfproduktes in die Reaktionen, ist nicht nur ein einfaches Verfahren, sondern überraschenderweise auch sehr kostengünstig. Diese Lösung des Problems war umso überraschender, da die Wirkung der Druckdestillation zum Teilproblem der DMC-Methanol zwar bekannt, aber als nicht nutzbar eingestuft worden war und nach dem Stand der Technik die Hilfe eines Extraktionsmittels oder eine Kristallisation mit anschließender Filtration und Destillation erforderlich ist. Denn durch die beschriebene Druckdestillation des DMC-Methanol-Gemisches ließ sich das DMC-Methanol-Wasser-Gemisch, wie es bei der Reaktion anfällt, nicht aufarbeiten.

Die vorliegende Erfindung betrifft ein Verfahren zur Herstellung von Dimethylcarbonat durch Umsetzung von Methanol mit Sauerstoff und Kohlenmonoxid in Gegenwart eines im Reaktionsmedium suspendierten oder gelösten kupferhaltigen Katalysators bei erhöhtem Druck und erhöhter Temperatur, Abtrennung des Katalysators und Aufarbeitung der Reaktionslösung, das dadurch gekennzeichnet ist, daß die katalysatorfreie Lösung in einer ersten Destillationskolonne zunächst vom Reaktionswasser als Sumpfprodukt befreit wird und das aus Dimethylcarbonat und Methanol bestehende Kopfprodukt in einer nachgeschalteten Druckdestillation bei erhöhter Temperautur und erhöhtem Druck in ein Sumpfprodukt aus reinem Dimethylcarbonat und ein an Dimethylcarbonat verarmtes, methanolisches Kopfprodukt getrennt wird und das Kopfprodukt in das Verfahren zurückgeführt wird.

Im erfindungsgemäßen, bevorzugt kontinuierlich durchgeführten Verfahren wird das umzusetzende Methanol im Reaktor in Gegenwart des Katalysators mit den Reaktionsgasen Sauerstoff und Kohlenmonoxid und evtl. zusätzlich einem Intertgas, in Berührung gebracht.

Im erfindungsgemäßen Verfahren werden als Katalysatoren Kupferverbindungen auf der Basis von Kupfer(I)- und/oder Kupfer(II)- salzen verwendet. Da es sich bei der Reaktion um eine Redoxreaktion handelt, liegen während der Umsetzung beide Kupferionenspezies vor. Als Kupferkatalysatoren werden vorzugsweise Kupfer(I)-halogenide, Kupfer(I)-acetylacetonat, Kupfer(I)-sulfat und/oder Kupfer(II)-alkoxyhalogenide, Kupfer(II)-alkoxysulfat, Kupfer(II)-alkoxyacetylacetonat, besonders bevorzugt wird Kupfer(II)-methoxychlorid eingesetzt. Das flüssige Reaktionsmedium besteht im wesentlichen aus dem umzusetzenden Methanol. Im allgemeinen beträgt das bezüglich des Methanolgehaltes auf 1 normierte Molverhältnis von Methanol:Dimethylcarbonat:Kupfer (Kupfer aus dem im Reaktionsmischung suspendierten und/oder gelösten Katalysator) im kontinuierlichen Betrieb im Reaktor 1:(0,005-1): (0,001-5), vorteilhaft 1:(0,02-0,5): (0,005-1) und besonders bevorzugt 1: (0,04-0,3): (0,01-0,16).

Die Umsetzung der Reaktionsgase mit dem Methanol wird bei einer Temperatur von 60 bis 200°C, bevorzugt von 80 bis 140°C, besonders bevorzugt von 100 bis 130°C durchgeführt. Die Reaktion wird bei Drücken von 1 bis 60 bar, bevorzugt bei 10 bis 40 und besonders bevorzugt bei 15 bis 35 bar durchgeführt. Der Druck wird zweckmäßigerweise durch Aufpressen der Reaktionsgase erzeugt.

Der dem Reaktor zugeführte Gasstrom kann in weiten Grenzen variiert werden, es wird jedoch zweckmäßigerweise ein Gesamtgasstrom, bestehend aus CO, Sauerstoff und evtl. einem Inertgas (wie z.B.: N₂, CO₂ usw.), bezogen auf das in der Reaktionslösung vorliegende Kupfer des Katalysators, von 0,2-100 NL/h und g Cu, bevorzugt 0,6 bis 80 NL/h und g Cu, besonders bevorzugt von 0,8 bis 5 NL/h und g Cu eingesetellt.
Die Zusammensetzung der Reaktionsgase Kohlenmonoxid und Sauerstoff kann in weiten Konzentrationsgrenzen variiert werden, es wird jedoch zweckmäßigerweise ein CO:O₂-Molverhältnis (normiert auf CO) von 1:(0,005-1,0) und bevorzugt von 1:(0,02-0,5) eingestellt. Der Sauerstoffpartialdruck ist bei diesen Molverhältnissen groß genug, um hohe Raum-Zeit-Ausbeuten erreichen zu können und gleichzeitig keine explosionsfähigen Kohlenmonoxid/Sauerstoff-Gasgemische bilden zu können. Die Reaktionsgase unterliegen keinen besonderen Reinheitsanforderungen, so kann Synthesegas als CO-Quelle und Luft als O₂-Träger dienen, es ist jedoch darauf zu achten, daß keine Katalysatorgifte wie z.B.: Schwefel oder dessen Verbindungen eingetragen werden.
Die Umsetzung des katalysatorhaltigen Methanols bei Reaktionsbedingungen mit den Reaktionsgasen wird zweckmäßigerweise bei einer möglichst niedrigen Konzentration des zwangsläufig anfallenden Reaktionswassers in der Reaktionsmischung durchgeführt, um Nebenreaktionen, wie die Bildung von Kohlendioxid und die gleichzeitige Desaktivierung des Kupferkatalysators, zu vermeiden. Die Reaktionswasserkonzentration beträgt im allgemeinen maximal 8 Gew.%, vorteilhaft maximal 6 Gew.%, bezogen auf die flüssige Phase.

Die Umsetzung wird bis zu einem gewünschten und einstellbaren Wert, bezogen auf eingesetztes Methanol von kleiner 35 % und besonders bevorzugt von kleiner 25 % betrieben.

Im erfindungsgemäßen Verfahren kann die Abtrennung des Katalysators auf verschiedenen Wegen erfolgen.

In einer bevorzugten Ausführungsform wird der Katalysator zusammen mit der Reaktionslösung kontinuierlich aus dem Reaktor entnommen und, wie in DE-OS 4203796 beschrieben, durch Sedimentation abgetrennt und die katalysatorfreie Reaktionslösung der Aufarbeitung zugeführt.

In einer weiteren bevorzugten Ausführungsform des erfindungsgemäßen Verfahrens wird die Reaktionslösung mit Hilfe des überschüssigen Reaktionsgases, wie in EP 0460732 A1, EP 0460735 A2 und DE-OS 3926709 A1 beschrieben, kontinuierlich aus dem Reaktor abgestrippt und so nach anschließender Kondensation katalysatorfrei erhalten.

Die erfindungsgemäß aufarbeitbaren Reaktionslösungen bestehen zu 50 bis 90 Gew.%, bevorzugt zu 55 bis 80 Gew.% und besonders bevorzugt zu 60 bis 75 Gew.% aus Methanol; zu 8 bis 45 Gew.%, bevorzugt 15 bis 40 Gew.% und besonders bevorzugt zu 20 bis 37 Gew.% aus Dimethylcarbonat. Der Wassergehalt dieser Reaktionslösungen liegt zwischen 0,5 und 15 Gew.%, bevorzugt zwischen 1 und 10 Gew.%, besonders bevorzugt zwischen 1,5 und 8 Gew.%. Alle Angaben sind auf das Gesamtgewicht der Reaktionslösung bezogen.

Erfindungsgemäß werden die so erhaltenen katalysatorfreien Reaktionslösungen durch einfache Rektifikation zunächst vom Reaktionswasser befreit. Die Rektifikation wird zunächst bei 0,1 bis 8 bar, bevorzugt 0,5 bis 4 bar und besonders bevorzugt bei 0,8 bis 3 bar durchgeführt. Der Kolonnensumpf wird hierbei auf 65 bis 200°C, bevorzugt 80 bis 160°C und besonders bevorzugt auf 90-150°C aufgeheizt.
Als Kopfprodukt erhält man DMC und Methanol und als Sumpfprodukt Wasser. Entgegen der bekannten Literatur wurde kein Azeotrop DMC/MeOH/H₂O bzw. DMC/H₂O erhalten, sondern eine Trennung des DMC und Methanol vom Reaktionswasser erreicht.
Aufgrund der bei der Reaktion angestrebten Zusammensetzung der Reaktionslösung wird bei der Destillation zunächst selektiv DMC/MeOH als Azeotrop und danach MeOH, das mit Wasser kein Azetrop bildet, abdestilliert.

In der bevorzugten Ausführungsform der Trennung wird als Kopfprodukt ein Gemisch aus DMC und MeOH und als Sumpfprodukt Wasser erhalten. Das aus DMC und MeOH bestehende Kopfprodukt besitzt noch einen Wassergehalt von kleiner 1 Gew.% und bevorzugt kleiner 0,1 Gew.% Wasser.

Zur Trennung von DMC und MeOH wird das DMC/MeOH-Gemisch dann in einer Druckkolonne destillativ, ohne Zusatz eines Hilfstoffes, in reines DMC als Sumpfprodukt und ein an DMC verarmtes, methanolisches Kopfprodukt aufgetrennt. Das so erhaltene DMC besitzt bereits eine so hohe Reinheit, daß es für die meisten Anwendungen ohne weitere Reinigung einsetzbar ist.
Die Rektifikation wird bei 1 bis 30 bar, bevorzugt 4 bis 20 bar und besonders bevorzugt bei 8 bis 15 bar durchgeführt. Der Kolonnensumpf wird hierbei auf 65 bis 250°C, bevorzugt 100 bis 220°C und besonders bevorzugt auf 130 bis 200°C aufgeheizt.

Das Kopfprodukt der Druckkolonne wird in das Verfahren zurückgeführt. Hierzu kann in der bevorzugten Ausführungsform des erfindungsgemäßen Verfahrens dieses Kopfprodukt, gegebenenfalls zusammen mit frischem Methanol, wieder dem Reaktor zugeführt werden.

In einer weiteren Ausführungsform wird dieses Kopfprodukt der Druckkolonne der ersten, bei Normaldruck arbeitenden Kolonne, an geeigneter Stelle wieder zugeführt.

In einer noch weiteren Ausführungsform kann das Kopfprodukt der Druckdestillation vor der Recyclisierung zum Reaktor einer weiteren Aufarbeitungskolonne zugeführt werden. In dieser weiteren Aufarbeitungskolonne wird als Kopfprodukt das Azeotrop aus Dimethylcarbonat und Methanol erhalten und an entsprechender Stelle der Druckkolonne wieder zugeführt. Als Sumpfprodukt wird Methanol mit mehr als 90 Gew.%, bevorzugt mehr als 95 Gew.%, besonders bevorzugt mehr als 98 Gew.% Methanol erhalten und dem Reaktor wieder zugeführt.

Als Destillationskolonnen für diese Trennvorgänge sind Kolonnen mit festen Einbauten, Füllkörpern und Packungen geeignet. Die zu verwendenden Füllkörper bzw. geordneten Packungen sind die für Destillationen an sich üblichen, wie sie beispielsweise in Ullmanns Encyclopädie der Techn. Chemie, 4. Auflage, Band 2, S. 528ff beschrieben sind. Beispielsweise seien genannt: Raschig- oder Pallringe, Berl-, Intalex- oder Torussättel, Interpackkörper. Diese Füllkörper können aus verschiedenen Materialien, wie Glas, Steinzeug, Porzellan, Edelstahl, Kunststoff und Metall bestehen, die insbesondere bei der Verwendung von Metall, gewebe-oder maschenartig verarbeitet sein können. Bevorzugte Füllkörper oder Packungen sind beispielsweise Pall- und Novolaxringe, Berlsättel, BX-Packungen, Montz-Pak, Metall-pak, Melladur, Kerepak und CY-Packungen.

Als Bodenkolonnen sind z.B. Sieb-, Glocken-, Ventil-, Tunnel- und Zentrifugalböden geeignet, die wiederum in unterschiedlichen Ausführungen vorliegen können.

Für die Kolonne zur Wasserabtrennung sind Kolonnen mit Füllkörpern oder Packungen besonders geeignet. Die Anzahl der theoretischen Böden liegt bei 1 bis 200, bevorzugt 5 bis 100, besonders bevorzugt 10 bis 60.

Für die Druckdestillationskolonne sind sowohl Boden- als auch Füllkörper-oder Packungskolonnen geeignet. Die Anzahl der theorethischen Böden liegt bei 1 bis 200, bevorzugt 5 bis 100, besonder bevorzugt 10 bis 60.

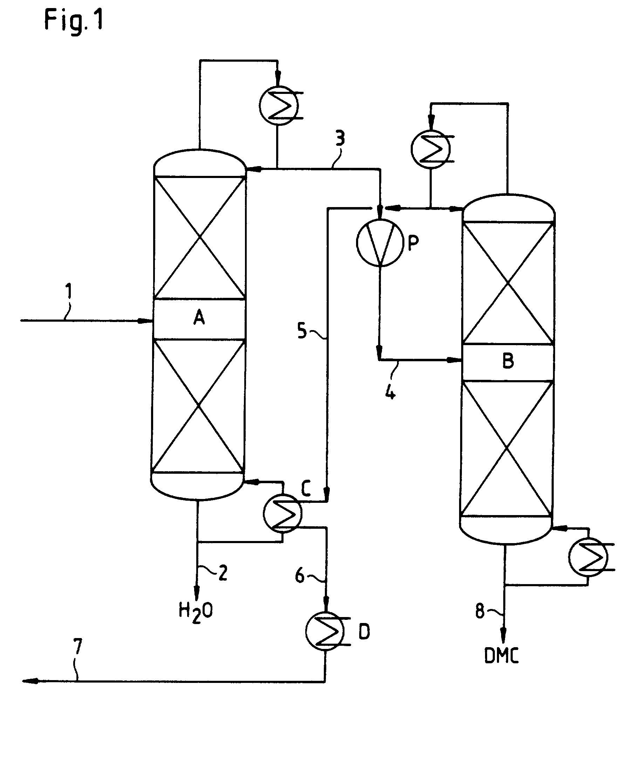

Figur 1 zeigt beispielsweise das erfindungsgemäße Verfahren:

Über Leitung 1 wird die katalysatorfreie Reaktionslösung, bestehend aus Methanol, Dimethylcarbonat und Wasser, in das mittlere Drittel der Kolonne A eingeleitet. Über Leitung 2 verläßt das abgetrennte Reaktionswasser als Sumpfprodukt produkt die Kolonne. Das aus Dimethylcarbonat und Methanol bestehende wird über Leitung 3, Pumpe P und Leitung 4 in das mittlere Drittel der Druckkolonne B eindosiert. Über Leitung 5 verläßt das an Dimethylcarbonat abgereicherte, methanolisches Kopfprodukt die Druckkolonne und heizt über Wärmetauscher C den Sumpf der Kolonne A. Nach Verlassen des Wärmetauschers C wird das Kopfprodukt gegebenenfalls im Wärmetauscher D weiter abgekühlt und über Leitung 7 in die Reaktion zurückgeführt. Das Wertprodukt Dimethylcarbonat verläßt über Leitung 8 die Kolonnen B und ist für die meisten Anwendungsfälle von genügender Reinheit.

Die Erfindung wird durch folgendes Beispiel näher erläutert, ohne jedoch darauf beschränkt zu sein.

### Beispiel

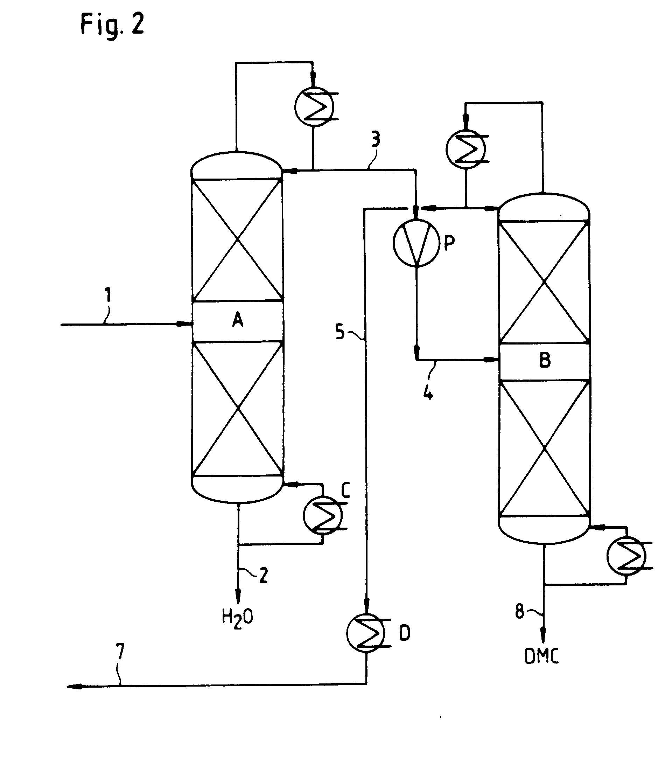

Es wurde eine Apparatur benutzt, wie sie in der Figur 2 dargestellt ist. Figur 2 unterscheidet sich von Figur 1 dadurch, daß Leitung 5 nicht mit dem Wärmeaustauscher C verschaltet ist. Die katalysatorfreie Reaktionslösung wurde in die Mitte der ersten Destillationskolonne A zudosiert. Diese Destillatonskolonne aus Glas (d=4,5 cm, 1 = 60 cm) zur Wasserabtrennung war mit 800 ml 4 x 4 mm V₄A-Maschendrahtwendeln gefüllt und besaß einen ölbeheizten Sumpfverdampfer mit Austrag und einen aufgesetzten Kondensator mit Rückflußteiler. Das Kopfprodukt von A wurde über ein Puffergefäß und eine Pumpe in die Mitte der Druckkolonne B eindosiert. Die Druckkolonne B war im Aufbau mit der Glaskolonne A identisch, jedoch mit dem Unterschied, daß sie aus Stahl gefertigt war und über ein Druckhaltesystem verfügte, das eine genaue Druckregelung erlaubte.
Die Apparatur wurde mit 560 g/h Reaktionsgemisch, bestehend aus 66 Gew.% Methanol, 31 Gew.% Dimethylcarbonat und 3 Gew.% Wasser, beschickt. Die Kolonne A arbeitete bei Normaldruck und hatte eine Sumpftemperatur von 100°C. Die Kolonne B wurde bei einem Druck von 10 bar betrieben und hatte eine Sumpftemperatur von 185°C.
Nach 6 h war die Apparatur im Gleichgewicht und lieferte pro Stunde 16,6 g Reaktionswasser als Sumpfablauf von A. Die Destillationskolonne B lieferte 75,5 g/h DMC mit einer Reinheit von 99,89 %, das Kopfprodukt von B betrug 469,4 g/h und bestand zu 79,1 Gew.% aus Methanol und zu 20,9 Gew.% aus DMC. Der Wassergehalt dieses Rückstroms betrug etwa 250 ppm.

## Patentansprüche

1. Verfahren zur Herstellung von Dimethylcarbonat durch Umsetzung von Methanol mit Sauerstoff und Kohlenmonoxid in Gegenwart eines im Reaktionsmedium suspendierten oder gelösten kupferhaltigen Katalysators bei erhöhtem Druck und bei erhöhter Temperatur, Abtrennung des Katalysators aund Aufarbeitung der Reaktionslösung, dadurch gekennzeichnet, daß die katalysatorfreie Reaktionslösung in einer ersten Destillationskolonne zunächst vom Reaktionswasser als Sumpfprodukt befreit wird und das aus Dimethylcarbonat und Methanol bestehende Kopfprodukt in einer nachgeschalteten Druckdestillation bei erhöhter Temperatur und erhöhtem Druck in ein Sumpfprodukt aus reinem Dimethylcarbonat und ein an Dimethylcarbonat verarmtes, methanolisches Kopfprodukt getrennt wird und das Kopfprodukt in das Verfahren zurückgeführt wird.

2. Verfahren nach Anspruch 1, dadurch gekennzeichnet, daß die katalysatorfreie Reaktionslösung, bezogen auf ihr Gesamtgewicht, zu 50-90 Gew.-%, bevorzugt zu 55-80 Gew.-%, besonders bevorzugt zu 60-75 Gew.-% aus Methanol, zu 8-45 Gew.-%, bevorzugt zu 15-40 Gew.-%, besonders bevorzugt zu 20-37 Gew.-% aus Dimethylcarbonat und zu 0,5-15 Gew.-%, bevorzugt zu 1-10 Gew.-%, besonders bevorzugt zu 1,5-8 Gew.-% aus Wasser besteht.

3. Verfahren nach Anspruch 1, dadurch gekennzeichnet, daß man die erste Destillationskolonne zur Wasserabtrennung bei 0,1 bis 8 bar, bevorzugt bei 0,5 bis 4 bar und besonders bevorzugt bei 0,8 bis 3 bar durchgeführt und den Kolonnensumpf hierzu auf 65 bis 200°C, bevorzugt 80 bis 160°C und besonders bevorzugt auf 90 bis 150°C aufheizt.

4. Verfahren nach Anspruch 1 bis 3, dadurch gekennzeichnet, daß man das Kopfprodukt der ersten Destillationskolonne in eine zweiten Destillationskolonne einleitet und bei 1 bis 20 bar, bevorzugt 4 bis 20 bar, besonders bevorzugt bei 8 bis 15 bar und einer Kolonnensumpftemperatur von 65 bis 250°C, bevorzugt 100 bis 220°C und besonders bevorzugt 130 bis 200°C trennt und als Sumpfprodukt reines DMC entnimmt.

5. Verfahren nach Anspruch 1 bis 4, dadurch gekennzeichnet, daß man das Kopfprodukt der zweiten Kolone in den Reaktor zurückführt.

6. Verfahren nach Anspruch 1 bis 5, dadurch gekennzeichnet, daß als Destillationskolonnen zur Wasserabtrennung, Kolonnen mit Füllkörpern oder Packungen eingesetzt werden, wobei die Anzahl der theoretischen Böden bei 1 bis 200, bevorzugt 5 bis 100, besonders bevorzugt 10 bis 60 liegt.

7. Verfahren nach Anspruch 1 bis 5, dadurch gekennzeichnet, daß als Destillationskolonnen für die Druckdestillation Boden-, Füllkörper-oder Packungskolonnen eingesetzt werden, wobei die Anzahl der theoretischen Böden zwischen 1 bis 200, bevorzugt 5 bis 100, besonders bevorzugt 10 bis 60 liegt.

## Claims

1. Process for the preparation of dimethyl carbonate by reaction of methanol with oxygen and carbon monoxide in the presence of a copper-containing catalyst suspended or dissolved in the reaction medium under increased pressure at elevated temperature, removal of the catalyst and working-up of the reaction solution, characterized in that the catalyst-free reaction solution is initially freed from the water of reaction as the bottom product in a first distillation column and the top product, comprising dimethyl carbonate and methanol, is separated in a subsequent pressure distillation at elevated temperature under increased pressure into a bottom product of pure dimethyl carbonate and a dimethyl carbonate-depleted, methanolic top product, and the top product is recycled to the process.

2. Process according to Claim 1, characterized in that the catalyst-free reaction solution comprises, based on its total weight, methanol to the extent of 50-90% by weight, preferably to the extent of 55-80% by weight, particularly preferably to the extent of 60-75% by weight, dimethyl carbonate to the extent of 8-45% by weight, preferably to the extent of 15-40% by weight, particularly preferably to the extent of 20-37% by weight, and water to the extent of 0.5-15% by weight, preferably to the extent of 1-10% by weight, particularly preferably to the extent of 1.5-8% by weight.

3. Process according to Claim 1, characterized in that the first distillation column for removal of the water is operated under 0.1 to 8 bar, preferably under 0.5 to 4 bar and particularly preferably under 0.8 to 3 bar, and for this the bottom of the column is heated up to 65 to 200°C, preferably 80 to 160°C and particularly preferably to 90 to 150°C.

4. Process according to Claims 1 to 3, characterized in that the top product of the first distillation column is introduced into a second distillation column and separated under 1 to 20 bar, preferably 4 to 20 bar, particularly preferably under 8 to 15 bar, at a column bottom temperature of 65 to 250°C, preferably 100 to 220°C and particularly preferably 130 to 200°C, and pure DMC is removed as the bottom product.

5. Process according to Claims 1 to 4, characterized in that the top product of the second column is recycled to the reactor.

6. Process according to Claims 1 to 5, characterized in that columns with fillings or packings are employed as distillation columns for removal of the water, the number of theoretical plates being 1 to 200, preferably 5 to 100, particularly preferably 10 to 60.

7. Process according to Claims 1 to 5, characterized in that tray, filled or packed columns are employed as distillation columns for the pressure distillation, the number of theoretical plates being between 1 and 200, preferably 5 to 100, particularly preferably 10 to 60.

## Revendications

1. Procédé pour la préparation de diméthylcarbonate par mise en réaction de méthanol avec de l'oxygène et du monoxyde de carbone en présence d'un catalyseur contenant du cuivre dissous ou mis en suspension dans le milieu de réaction, sous pression élevée et à température élevée, par séparation du catalyseur et traitement de la solution réactionnelle, caractérisé en ce qu'on libère la solution réactionnelle exempte de catalyseur dans une première colonne de distillation d'abord de l'eau réactionnelle sous forme de produit de bas de colonne et on sépare le produit de tête constitué par du diméthylcarbonate et par du méthanol dans une distillation sous pression prévue à la suite, à température élevée et sous pression élevée, en un produit de bas de colonne constitué de diméthylcarbonate pur et en un produit de tête méthanolique appauvri en diméthylcarbonate, et on renvoie le produit de tête dans le procédé.

2. Procédé selon la revendication 1, caractérisé en ce que la solution réactionnelle exempte de catalyseur, rapportée à son poids total, est constituée, à concurrence de 50 à 90% en poids, de préférence de 55 à 80% en poids, de manière particulièrement préférée de 60 à 75% en poids, par du méthanol; à concurrence de 8 à 45% en poids, de préférence de 15 à 40% en poids, de manière particulièrement préférée de 20 à 37% en poids, par du diméthylcarbonate; et à concurrence de 0,5 à 15% en poids, de préférence de 1 à 10% en poids, de manière particulièrement préférée de 1,5 à 8% en poids par de l'eau.

3. Procédé selon la revendication 1, caractérisé en ce qu'on effectue la première distillation en colonne pour la séparation de l'eau sous une pression de 0,1 à 8 bar, de préférence de 0,5 à 4 bar, de manière particulièrement préférée de 0,8 à 3 bar, et on chauffe à cet effet le produit de bas de colonne à une température de 65 à 200°C, de préférence de 80 à 160°C, de manière particulièrement préférée de 90 à 150°C.

4. Procédé selon les revendications 1 à 3, caractérisé en ce qu'on introduit le produit de tête de la première colonne de distillation dans une seconde colonne de distillation, on le sépare sous une pression de 1 à 20 bar, de préférence de 4 à 20 bar, de manière particulièrement préférée de 8 à 15 bar et à une température de produit de bas de colonne de 65 à 250°C, de préférence de 100 à 220°C, de manière particulièrement préférée de 130 à 200°C, et on prélève du DMC pur sous forme de produit de bas de colonne.

5. Procédé selon les revendications 1 à 4, caractérisé en ce qu'on renvoie le produit de tête de la seconde colonne dans le réacteur.

6. Procédé selon les revendications 1 à 5, caractérisé en ce qu'on met en oeuvre, à titre de colonnes de distillation pour la séparation de l'eau, des colonnes munies de corps de remplissage ou de garnissages, le nombre des plateaux théoriques s'élevant de 1 à 200, de préférence de 5 à 100, de manière particulièrement préférée de 10 à 60.

7. Procédé selon les revendications 1 à 5, caractérisé en ce qu'on met en oeuvre, à titre de colonnes de distillation pour la distillation sous pression, des colonnes à plateaux, des colonnes contenant des matières de remplissage ou encore des colonnes à garnissages, le nombre des plateaux théoriques s'élevant entre 1 et 200, de préférence de 5 à 100, de manière particulièrement préférée de 10 à 60.
